# EUROPÄISCHE PATENTSCHRIFT

(11) **EP 0 796 835 B1**
(45) Veröffentlichungstag und Bekanntmachung des Hinweises auf die Patenterteilung: **17.05.2000**
(21) Anmeldenummer: 97104444.1
(22) Anmeldetag: 15.03.1997
(51) Int. Cl.: C07C 45/36, C07C 409/16, C07C 29/50, C07C 27/12, B01J 31/02

(54) **Verfahren zur Oxidation isoalkyl-aromatischer Kohlenwasserstoffe und Katalysator zur Durchführung des Verfahrens**
Process for the oxidation of isoalkyl-aromatic hydrocarbons and catalyst for carrying out this process
Procédé pour l'oxydation d'hydrocarbures isoalkyl-aromatiques et catalyseur pour la mise en oeuvre de ce procédé

(30) Priorität: 20.03.1996 PL 31341896
(43) Veröffentlichungstag der Anmeldung: 24.09.1997
(73) Patentinhaber: Rütgers Kureha Solvents GmbH, 47138 Duisburg (DE)
(72) Erfinder: Zawadiak, Jan, 44-105 Gliwice (PL); Stec, Zbigniew, 44-100 Gliwice (PL); Knips, Ulrich, 59174 Kamen (DE); Zellerhoff, Robert, 46499 Hamminkeln (DE); Gilner, Danuta, 44-101-Gliwice (PL); Orlinska, Beata, 40-868 Katowice (PL); Polaczek, Jerzy, 02-784 Warszawa (PL)
(74) Vertreter: Cohausz & Florack

(56) Entgegenhaltungen:
- EP-A- 0 267 759
- GB-A- 309 005
- CHEMICAL ABSTRACTS, vol. 123, no. 19, 6.November 1995 Columbus, Ohio, US; abstract no. 256345, MAEDA T ET AL: "Method for producing acetophenone by oxidation of ethylbenzene" XP002033454 & JP 07 196 573 A (SHELL INT RESEARCH;NETH.) 1.August 1995
- CHEMICAL ABSTRACTS, vol. 114, no. 13, 1.April 1991 Columbus, Ohio, US; abstract no. 121726, ZAWADIAK J ET AL: "Preparation of tertiary alkylaromatic peroxides, particularly dicumyl peroxide, using alkylammonium halide additives" XP000187312 & BR 8 906 394 A (MAKEDONSKY, KONSTANTIN;BRAZIL) 21.August 1990
- REACT. KINET. CATAL. LETT. (RKCLAU,03044122);85; VOL.27 (2); PP.231-3, SLOVAK TECH. UNIV.;FAC. CHEM.; BRATISLAVA; 812 37; CZECH. (CS), XP002033453 HRONEC M ET AL: "The use of phase-transfer catalysis for the initiation of p-xylene oxidation"

## Beschreibung

Die Erfindung betrifft die katalytische Oxidation isoalkylaromatischer Kohlenwasserstoffe sowie Katalysator zur Durchführung dieser Reaktion.

Die Oxidation isoalkylaromatischer Kohlenwasserstoffe zu organischen Ketonen, Alkoholen und Peroxiden ist von großer industrieller Bedeutung und wird hauptsächlich zur Herstellung isoalkylaromatischer Hydroperoxide verwendet, deren Zersetzung zur Entstehung entsprechender hydroxyaromatischer Verbindungen führt. Es ist auch versucht worden, aus diesen Rohstoffen organische Peroxide zu gewinnen.

Aus der PL 156 813 ist ein Verfahren zur Gewinnung von Dicumylperoxid durch Umsetzung von Cumol mit Cumylhydroperoxid bekannt. Die JP 6 049 191 beschreibt die Gewinnung von Dicumylperoxid durch Kondensation des Cumylhydroperoxids mit 2-Phenyl-2-propanol. Aus der CZ 198 676 ist es bekannt, Dicumylperoxid durch unmittelbare Oxidation des Cumols mit Luft in Gegenwart von Eisencapronat bei einer Temperatur von 94°C zu gewinnen. Diese Umsetzung verläuft jedoch recht langsam. Das nach 65 Stunden erhaltene Produkt enthielt 21,7 % Dicumylperoxid, 12,5 % Acetophenon und 22,2 % 2-Phenyl-2-propanol.

In Neftekhimiya, Bd. 11, 1971, 862-866 wurde auch die Gewinnung von Bis-2-isopropylnaphthylperoxid, 2-Isopropylnaphthylalkohol mittels Sauerstoff in Gegenwart von Kupfer(II)-nitrat und Azodiisobutyronitril bei einer Temperatur von 95°C erörtert. Das nach 68 Stunden gewonnene Produkt enthielt 34,1 Mol% Bis-2-isopropylnaphthylperoxid, 9,4 Mol% 2-Isopropylnaphthylalkohol und 1,2 Mol% 2-Acetylnaphthalin.

Diese bekannten Verfahren kennzeichnet eine lange Reaktionsdauer von mehr als 10 Stunden und eine unzulängliche Umsetzung des eingesetzten Kohlenwasserstoffs, die gewöhnlich 60% nicht überschreitet. Die praktische und insbesondere industrielle Anwendbarkeit dieser Verfahren ist deshalb beschränkt.

Aus Chemical Abstracts 114:121726x ist ein Verfahren zur Herstellung von Dicumylperoxid bekannt, in dem Cumol in Gegenwart von Kupfer(II)-chlorid und Cetyltrimethylammoniumbromid bei 70°C unter Stickstoffatmosphäre mit Cumolhydroperoxid umgesetzt wird. Aus Hronec et al., React. Kinet. Catal. Lett., Vol. 27, Nr. 2, S. 231-233 (1985) ist ferner die Oxidation von p-Xylol zu p-Cresol durch molekularen Sauerstoff in Gegenwart von Cobaltbromid und einem quaternären Oniumsalz bekannt.

Gelöst wird diese Aufgabe durch die katalytische Oxidation isoalkylaromatischer Kohlenwasserstoffe, die ein tertiäres Kohlenstoffatom in α-Stellung zum aromatischen Ring aufweisen, zu Ketonen, Alkoholen und Peroxiden, wobei die isoalkylaromatischen Kohlenwasserstoffe in flüssiger Phase bei einer Temperatur von 60 bis 120°C durch Sauerstoff in Gegenwart eines Kupfer-, Cobalt- oder Mangansalzes und Alkylammoniumsalz oder Alkylphosphoniumsalz oxidiert werden.

Die Erfindung betrifft ferner einen Katalysator für die Durchführung dieser Oxidation, der ein Mangansalz und ein Alkylammoniumsalz oder ein Alkylphosphoniumsalz enthält.

Erfindungsgemäß geeignete Anionen dieser Mangansalze sind Chloride, Bromide, Jodide, Nitrate, Sulfate, Hydrogensulfate oder Sulfonate. Ferner kann das Anion ein Fettsäureanion wie Steärat, Palmitat und Myristat sein.

Die erfindungsgemäß bevorzugten Alkylammoniumsalze und Alkylphosphoniumsalze sind solche, die vorzugsweise mindestens einen Alkylrest mit 3 bis 20 Kohlenstoffatomen aufweisen und worin das Anion ein Chlorid, Bromid, Jodid, Nitrat, Sulfat, Hydrogensulfat oder Sulfonat sein kann.

Das Molverhältnis Metallsalz unterschiedlicher Valenz zu Alkylammoniumsalz oder Alkylphosphoniumsalz beträgt 0,5 bis 50 zu 1.

Erfindungsgemäß einzusetzende Reaktanten sind Isoalkylaromaten der allgemeinen Formel Ar-CHRR', worin R und R' gleich oder verschieden sind und Methyl, Ethyl oder Propyl bedeuten. Ar bedeutet Phenyl und seine Homologen sind beispielsweise Toluyl, Benzyl oder Xylyl sowie durch einen oder mehrere Ethylreste substituiertes Phenyl. Homologe von Naphtyl sind dessen Methylderivate und Ethylderivate.

Bevorzugte Reaktanten des erfindungsgemäßen Verfahrens sind Isopropylaromaten oder Isobutylaromaten wie Cumol, p-tert.-Butylcumol (TEK), Isopropylnaphthalin (IPN), Isobutylnaphthalin und 2,6-Diisopropylnaphthalin. Voraussetzung für die Durchführung des erfindungsgemäßen Verfahrens ist das Vorhandensein eines tertiären Kohlenstoffatoms in alpha-Stellung zum aromatischen Ring.

Wird 2-Isopropylnaphtalin als Reaktant eingesetzt, ist das gleichzeitige Vorhandensein von 1-Isopropylnaphtalin unkritisch.

Die Reaktanten werden bei 60 bis 120°C in Gegenwart des Katalysators umgesetzt, wobei der Katalysator dem flüssigen Reaktanten zugesetzt werden kann. Bei der Umsetzungstemperatur liegen die Reaktanten in flüssiger Form vor. Das Molverhältnis Reaktant zu Metallsalz beträgt im erfindungsgemäßen Verfahren vorzugsweise 4000 : 1 bis 80 : 1. Das Molverhältnis Metallsalz unterschiedlicher Valenz zu Alkylammoniumsalz oder Alkylphosphoniumsalz beträgt vorzugsweise 0,5 bis 50 zu 1. Das Verfahren erfolgt drucklos. Die Reaktionsdauer beträgt 2 bis 20 Stunden.

Verfahrensprodukte des erfindungsgemäßen Verfahrens sind Arylalkylketone der allgemeinen Formel Ar-CO-R, Arylalkylcarbinole der allgemeinen Formel Ar-CRR'OH und Di(arylalkylperoxide) der allgemeinen Formel Ar-CRR'-O-O-CRR'-Ar, worin Ar, R und R' die oben genannte Bedeutung haben. Beispiele der erfindungsgemäß erhaltenen Ketone sind Acetonaphton, Acetophenon und 2,6-Diacetylnaphtalin. Beispiele erfindungsgemäß erhaltener Arylalkylcarbinole sind 2-(2-Naphtyl)isopropanol und Cumylalkohol.

Beispiele der erfindungsgemäß erhaltenen Diarylalkylperoxide sind tertiäre symetrische Bis[1-alkyl-1-(1- oder 2-naphthyl)alkyl]peroxide.

In Abhängigkeit von den gewählten Oxidationsbedingungen lassen sich mit dem erfindungsgemäßen Verfahren in kurzer Zeit eine Konzentration organischer Peroxide von etwa 20 Gew.% bzw. Alkohol- und Ketonkonzentrationen von über 45 Gew.% im Produktgemisch erzielen. Diese Produkte sind wertvolle Zwischenprodukte für organische Synthesen mittels Veresterungen, Dehydrierungen und Oxidationen. Organische Peroxide sind einsetzbar als Initiatoren und Vernetzungskatalysatoren bei der Herstellung von Kautschuken.

Die folgenden Beispiele dienen der Erläuterung der Erfindung.

### Beispiel 1

In einen Glasreaktor mit einem Volumen von 40 cm³, der mit einem Rührwerk, einem Rückflußkühler, einer Einperldrossel, einem Thermometer und einem Heizmantel ausgestattet ist, werden 0,28 Mol Cumol, 2,56 x 10⁻⁴ Mol Kupfer(II)-chloriddihydrat und 5,36 x 10⁻⁶ Mol Tetrabutylammoniumbromid (TBAB) geleitet und das Reaktionsgemisch dann bis auf 110°C unter Einwirkung von molekularem Sauerstoff erwärmt, der mit einer Geschwindigkeit von 15 dm³/h zwölf Stunden lang durchgeleitet wird. Das Endprodukt enthält 32,5 Gew.% Dicumylperoxid, 18,6 Gew.% 2-Phenyl-2-propanol und 23,6 Gew.% Acetophenon.

Die Beispiele 2 bis 11 werden in gleicher Weise durchgeführt. Ihre Reaktionsbedingungen und Ergebnisse sind in der folgenden Tabelle wiedergegeben.

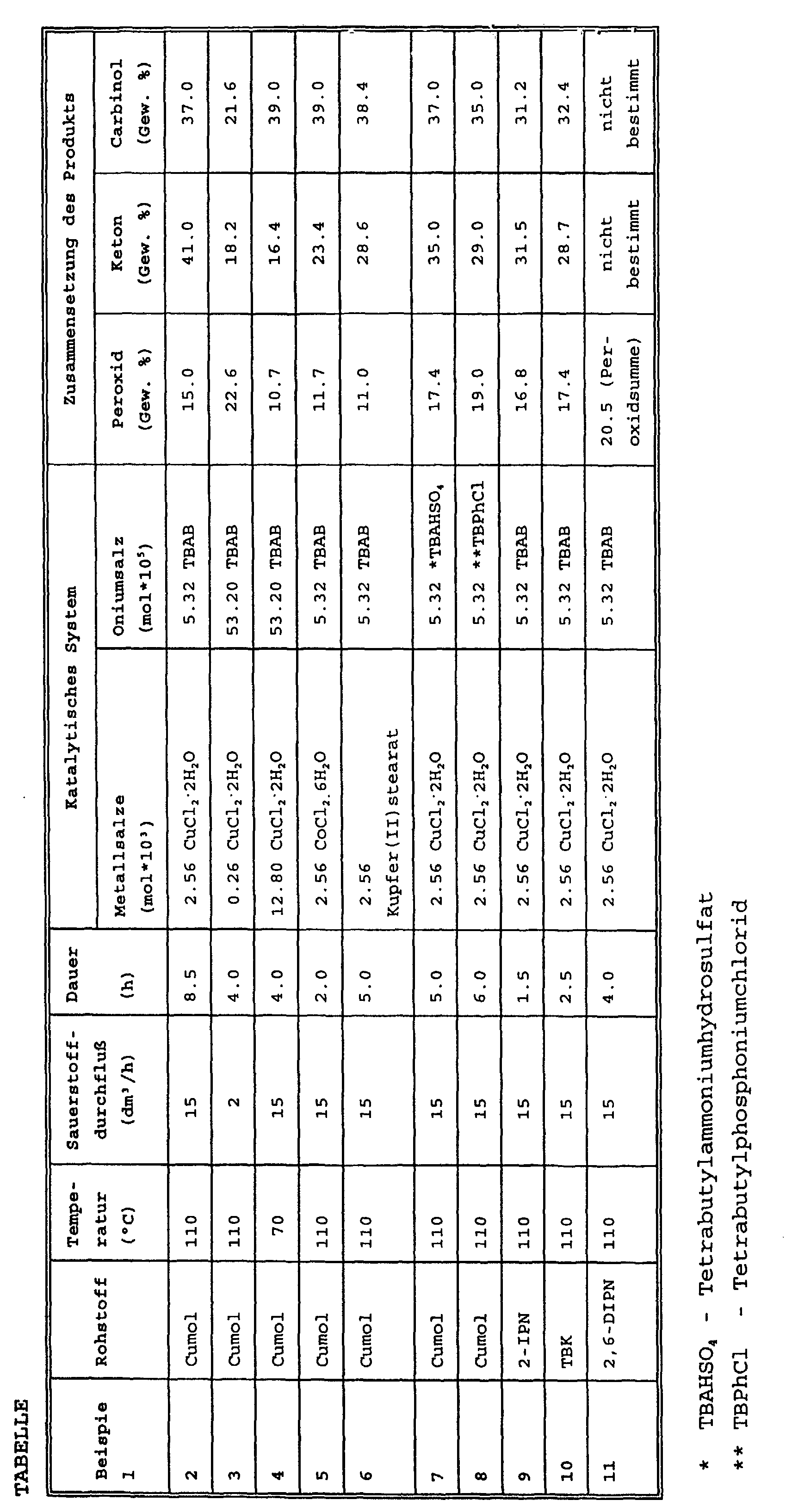

## Patentansprüche

1. Verfahren zur katalytischen Oxidation isoalkylaromatischer Kohlenwasserstoffe, die ein tertiäres Kohlenstoffatom in α-Stellung zum aromatischen Ring aufweisen, zu Ketonen, Alkoholen und Peroxiden, dadurch gekennzeichnet, daß man die isoalkylaromatischen Kohlenwasserstoffe in flüssiger Phase bei einer Temperatur von 60 bis 120°C durch Sauerstoff in Gegenwart eines Kupfer-, Cobalt- oder Mangansalzes und Alkylammoniumsalz oder Alkylphosphoniumsalz oxidiert.

2. Verfahren nach Anspruch 1, dadurch gekennzeichnet, daß das Alkylammoniumsalz oder Alkylphosphoniumsalz mindestens einen Alkylrest mit 3 bis 20 Kohlenstoffatomen aufweist.

3. Katalysator für die Oxidation isoalkylaromatischer Kohlenwasserstoffe, enthaltend ein Mangansalz und ein Alkylammoniumsalz oder Alkylphosphoniumsalz in einem Verhältnis Mangansalz zu Alkylammoniumsalz oder Alkylphosphoniumsalz von 0,5 bis 50 zu 1.

4. Katalysator nach Anspruch 3, dadurch gekennzeichnet, daß das Alkylammoniumsalz oder Alkylphosphoniumsalz mindestens einen Alkylrest mit 3 bis 20 Kohlenstoffatomen aufweist.

5. Verwendung eines Gemischs enthaltend ein Kupfer-, Cobalt- oder Mangansalz und ein Alkylammonium- oder Alkylphosphoniumsalz als Katalysator zur Oxidation isoalkylaromatischer Kohlenwasserstoffe, die ein tertiäres Kohlenstoffatom in α-Stellung zum aromatischen Ring aufweisen, zu Ketonen, Alkoholen und Peroxiden in flüssiger Phase bei einer Temperatur von 60 bis 120°C.

## Claims

1. Process for the catalytic oxidation of isoalkylaromatic hydrocarbons into ketones, alcohols and peroxides, comprising the oxidation of the isoalkylaromatic hydrocarbons in the fluid phase at a temperature of 60 to 120°C by oxygen in the presence of a catalyst composed of a metal salt having a different valency, and of an alkylammonium salt or an alkylphosphonium salt.

2. Process according to claim 1, wherein the alkylammonium salt or alkylphosphonium salt has at least one alkyl residue with 3 to 20 carbon atoms.

3. Catalyst for the oxidation of isoalkylaromatic hydrocarbons, containing a manganese and a alkylammonium salt or alkylphosphonium salt in a molar ratio of metal salt to alkylammonium salt or an alkylphosphonium salt or an alkylphosphonium salt of 0.5 to 50:1.

4. Catalyst according to claim 3, wherein the alkylammonium salt or alkylphosphonium salt has at least one alkyl residue with 3 to 20 carbon atoms.

5. Use of a mixture containing a copper, cobalt- or manganese salt and an alkylammonium or alkylphosphonium salt as catalyst for the oxidation of isoalkyl aromatic carbon hydrates, bearing a tertiary hydrogen atom in alpha-position to the aromatic cycle, to ketones, alcohols and peroxides in liquid phase at a temperature of 60 to 120°C.

## Revendications

1. Procédé pour l'oxydation catalytique d'hydrocarbures isoalkyl-aromatiques qui présentent un atome de carbone tertiaire en position α par rapport au cycle aromatique, en cétones, alcools et peroxydes, caractérisé en ce que l'on oxyde les hydrocarbures isoalkyl-aromatiques en phase liquide à une température de 60 à 120°C par l'oxygène en présence d'un sel de cuivre, de cobalt ou de manganèse et d'un sel d'alkylammonium ou d'un sel d'alkylphosphonium.

2. Procédé selon la revendication 1, caractérisé en ce que le sel d'alkylammonium ou le sel d'alkylphosphonuim présente au moins un radical alkyle ayant de 3 à 20 atomes de carbone.

3. Catalyseur pour l'oxydation d'hydrocarbures isoalkyl-aromatiques contenant un sel de manganèse et un sel d'alkylammonium ou un sel d'alkylphosphonium avec un rapport du sel de manganèse par rapport au sel d'alkylammonium ou au sel d'alkylphosphonium compris entre 0,5 et 50 par rapport à 1.

4. Catalyseur selon la revendication 3, caractérisé en ce que le sel d'alkylammonium ou le sel d'alkylphosphonium présente au moins un radical alkyle ayant de 3 à 20 atomes de carbone.

5. Utilisation d'un mélange contenant un sel de cuivre, de cobalt ou de manganèse et un sel d'alkylammonium ou un sel d'alkylphosphonium comme catalyseur pour l'oxydation des hydrocarbures isoalkyl-aromatiques, qui présentent un atome de carbone tertiaire en position α par rapport au cycle aromatique, en cétones, alcools et peroxydes en phase liquide à une température comprise entre 60 et 120°C.
